# EUROPEAN PATENT SPECIFICATION

(11) **EP 2 529 681 B1**
(45) Date of publication and mention of the grant of the patent: **07.03.2018**
(21) Application number: 12168303.1
(22) Date of filing: 16.05.2012
(51) Int. Cl.: A61B 17/295, A61B 18/14

(54) **Modular shaft for endoscopic vessel sealer and divider**
Modularer Schaft für endoskopischen Gefäßverschluss und Abscheider
Arbre modulaire pour vaisseau endoscopique de suture et de séparation

(30) Priority: 17.05.2011 US 201161487052 P
(43) Date of publication of application: 05.12.2012
(73) Proprietor: Covidien LP, Mansfield, MA 02048 (US)
(72) Inventor: Kappus, John J., Denver, CO Colorado 80218 (US); Gerhardt, Thomas J., Jr., Littleton, CO Colorado 80128 (US); Siebrecht, Wayne, Golden, CO Colorado 80403 (US); Johnson, Larry, Bennett, CO Colorado 80102 (US); Larson, Eric R., Boulder, CO Colorado 80301 (US); Hempstead, Russell D., Lafayette, CO Colorado 80026 (US); Hart, Keir, Lafayette, CO Colorado 80026 (US); Taylor, James H., Lafayette, CO Colorado 80026 (US)
(74) Representative: Soames, Candida Jane

(56) References cited:
- EP-A1- 2 090 245

## Description

### BACKGROUND

The present disclosure relates to an electrosurgical forceps and more particularly, the present disclosure relates to a modular shaft assembly for use with a variety of endoscopic bipolar electrosurgical forceps for sealing and/or cutting various tissue structures.

### Technical Field

Electrosurgical forceps utilize both mechanical clamping action and electrical energy to affect hemostasis by heating the tissue and blood vessels to coagulate, cauterize and/or seal tissue. Many surgical procedures require cutting and/or ligating large blood vessels and large tissue structures. Due to the inherent spatial considerations of the surgical cavity, surgeons often have difficulty suturing vessels or performing other traditional methods of controlling bleeding, e.g., clamping and/or tying-off transected blood vessels or tissue. By utilizing an elongated electrosurgical forceps, a surgeon can either cauterize, coagulate/desiccate and/or simply reduce or slow bleeding simply by controlling the intensity, frequency and duration of the electrosurgical energy applied through the jaw members to the tissue. Most small blood vessels, i.e., in the range below two millimeters in diameter, can often be closed using standard electrosurgical instruments and techniques. However, larger vessels can be more difficult to close using these standard techniques.

In order to resolve many of the known issues described above and other issues relevant to cauterization and coagulation, a recently developed technology has been developed called vessel or tissue sealing. The process of coagulating vessels is fundamentally different than electrosurgical vessel sealing. For the purposes herein, "coagulation" is defined as a process of desiccating tissue wherein the tissue cells are ruptured and dried. "Vessel sealing" or "tissue sealing" is defined as the process of liquefying the collagen in the tissue so that it reforms into a fused mass with limited demarcation between opposing tissue structures. Coagulation of small vessels is sufficient to permanently close them, while larger vessels and tissue need to be sealed to assure permanent closure.

In order to effectively seal larger vessels (or tissue) two predominant mechanical parameters are accurately controlled: 1) the pressure applied to the tissue (e.g., between about 3 kg/cm² to about 16 kg/cm²); and 2) the gap distance between the electrodes (e.g., between about 0.001 inches to about 0.008 inches (0.003cm to 0.020cm)). More particularly, accurate application of pressure is important to oppose the walls of the vessel; to reduce the tissue impedance to a low enough value that allows enough electrosurgical energy through the tissue; to overcome the forces of expansion during tissue heating; and to contribute to the end tissue thickness which is an indication of a good seal.

As a result thereof, providing instruments which consistently provide the appropriate closure force between opposing electrode within a particular pressure range and within a particular gap range will enhance the chances of a successful seal. However manufacturing a variety of instruments which consistently perform to these tight tolerance standards of gap and pressure typically require the manufacturer to customize the drive assemblies and cutting assemblies for each specific instrument type due to spatial limitations, ergonomics, end effector arrangements or other factors. As can be appreciated this adds to the overall cost of each instrument from an Research and development standpoint and from an assembly standpoint.

Reference is made to EP 2 090 245 A1. This document discloses a surgical stapling instrument, that can include a handle, a shaft extending from the handle, wherein the shaft defines an axis, and a disposable loading unit which is assembled to the shaft in a direction which is transverse to the shaft axis. Such a connection between the disposable loading unit and the shaft can prevent, or at least inhibit, the disposable loading unit from being unintentionally displaced proximally and/or distally relative to the shaft of the surgical instrument. The surgical stapling instrument and/or disposable loading unit can further include a threaded collar and/or detent assembly configured to hold the disposable loading unit in place. In various embodiments, a disposable loading unit can include a lockout feature which can prevent, or at least inhibit, an expended disposable loading unit from being reassembled to the elongated body of the surgical instrument.

### SUMMARY

The invention is defined by the independent claim below. Dependent claims are directed to optional features and preferred embodiments. The present disclosure relates to a modular shaft assembly for use with a variety of different endoscopic forceps each having a housing including a handle assembly and at least one moveable handle. The modular shaft assembly includes a shaft having proximal end distal ends and an end effector assembly that engages the distal end thereof. The end effector includes a pair of opposing jaw members that are movable relative to one another from an open position wherein the jaw members are disposed in spaced relation relative to one another to a closed position for grasping tissue therebetween. The modular shaft assembly also includes a universal drive assembly attached at the proximal end of the shaft. The universal drive assembly is operably engageable with various handle assemblies of the different endoscopic forceps such that actuation of the movable handle of any of the variety of different forceps causes the universal drive assembly to actuate the jaw members to move between the open position and closed positions.

Additionally or alternatively, a universal knife assembly may be operably engageable with a variety of knife actuators of the variety of different endoscopic forceps. The universal knife assembly may be selectively configurable with the variety of knife actuators to accommodate a variety of different knife stroke lengths of the universal knife assembly.

Additionally or alternatively, the distal end of the shaft may include a universal coupling that selectively engages a corresponding coupling on a variety of end effector assemblies having a variety of different jaw member configurations. The shaft may include a plurality of mechanical interfaces that are configured to mate with a corresponding common plurality of mechanical interfaces disposed within the variety of different endoscopic forceps. The plurality of mechanical interfaces disposed within the variety of different endoscopic forceps. The plurality of mechanical interfaces that are configured to mate with a corresponding common plurality of mechanical interfaces disposed within the variety of endoscopic forceps may be disposed on an outer periphery of the shaft. For example, the shaft may include a bushing disposed on the shaft that mates within a corresponding mechanical interface disposed within a distal end the housing.

Additionally or alternatively, the modular shaft assembly includes a universal rotating assembly that is configured to operably engage a variety of rotating actuators of the variety of different endoscopic forceps.

The present disclosure also relates to a modular shaft assembly for use with a variety of different endoscopic forceps that includes a shaft having proximal and distal ends and an end effector assembly including a pair of jaw members attached to the distal end of the shaft. A universal drive assembly is attached at the proximal end of the shaft that is operably engageable with a variety of different handle assemblies of the variety of different endoscopic forceps such that actuation thereof causes the universal drive assembly to actuate the jaw members of the end effector assembly. A universal knife assembly is included that has a knife with a variable stroke length configured for selective reciprocation between the jaw members. One or more links may be included that operably engage a variety of different knife actuators of the endoscopic forceps. The link(s) is(are) selectively positionable to vary the knife stroke length of the knife according to the dimensions of each endoscopic forceps.

Additionally or alternatively, two links may be selectively positionable relative to one another to vary the stroke length of the knife. One or more of the links may include a series of apertures defined therethrough that mechanically engage a corresponding pin to position the links relative to one another to vary the stroke length of the knife.

Additionally or alternatively, a coding system may be included that facilitates adjustment of the stroke length of the knife according to a particular endoscopic forceps. The coding system may include a series of numbers or letters, color-coded elements, indicia or symbols.

### BRIEF DESCRIPTION OF THE DRAWINGS

Various embodiments of the subject instrument are described herein with reference to the drawings wherein:
Fig. 1A is a perspective view of a bipolar forceps shown in open configuration and including a housing, a shaft, handle assembly, trigger assembly and an end effector assembly according to the present disclosure;
Fig. 1B is a perspective view of the bipolar forceps of Fig. 1A shown in closed configuration;
Figs. 2A - 2B are a rear, perspective views of the forceps of Figs. 1A and 1B, respectively;
Fig. 3A is an enlarged, top perspective view of a top jaw member of the end effector assembly of Fig. 1A with parts separated;
Fig. 3B is an enlarged, top perspective view of a bottom jaw member of the end effector assembly of Fig. 1A with parts separated;
Figs. 4A-4B are perspective views of the endoscopic forceps of Fig. 1A with the internal working components of the forceps exposed and showing actuation of the trigger assembly;
Fig. 5A is a greatly-enlarged, perspective view of the handle assembly in open configuration;
Fig. 5B is a greatly-enlarged, perspective view of the handle assembly in closed configuration;
Fig. 6 is an internal, side view of the endoscopic forceps of Fig. 1B with the trigger shown in an actuated position;
Fig. 7A is an enlarged, side cross-sectional view showing the end effector in a closed position and the knife in an unactuated position;
Fig. 7B is an enlarged, side cross-sectional view showing the end effector in a closed position and the knife in an actuated position;
Fig. 7C is an enlarged, front perspective view of a bottom jaw member of the end effector assembly showing the knife in an actuated position;
Fig. 8 is an exploded, perspective view of the forceps of Fig. 1A;
Fig. 9 is an enlarged, exploded perspective view of the housing;
Fig. 10 is an enlarged, exploded perspective view of the end effector assembly and the shaft;
Fig. 11 is a greatly enlarged, exploded perspective view of the end effector assembly;
Fig. 12 is a side, perspective view of an alternate embodiment of the present disclosure showing a modular shaft assembly for use with a variety of different forceps;
Figs. 13A - 13C are model internal views of the shaft assembly of Fig. 12 disposed within two different forceps;
Fig. 13D is a rear, perspective view of the shaft assembly of Fig. 12 coupled to a handle assembly and trigger assembly;
Fig. 14 is an enlarged rear, perspective view of a distal end of a forceps with a selectively engageable end effector assembly operably coupled thereto;
Figs. 15A-15B are enlarged, side views of an alternate shaft assembly for use with a variety of different forceps;
Fig. 15C is a side view of the shaft assembly of Fig. 15A shown disposed with a forceps having a first shaft diameter;
Fig. 15D is a side view of the shaft assembly of Fig. 15A shown disposed with a forceps having a second shaft diameter;
Fig. 16A is a rear, internal perspective view of a knife lockout assembly for use with a forceps shown in an engaged position;
Fig. 16B is a rear, internal perspective view of a knife lockout assembly for use with a forceps shown in a disengaged position; and
Fig. 17 is a rear, internal perspective view of an alternate knife lockout assembly having a variable stroke length for use with a forceps shown in a disengaged position.

### DETAILED DESCRIPTION

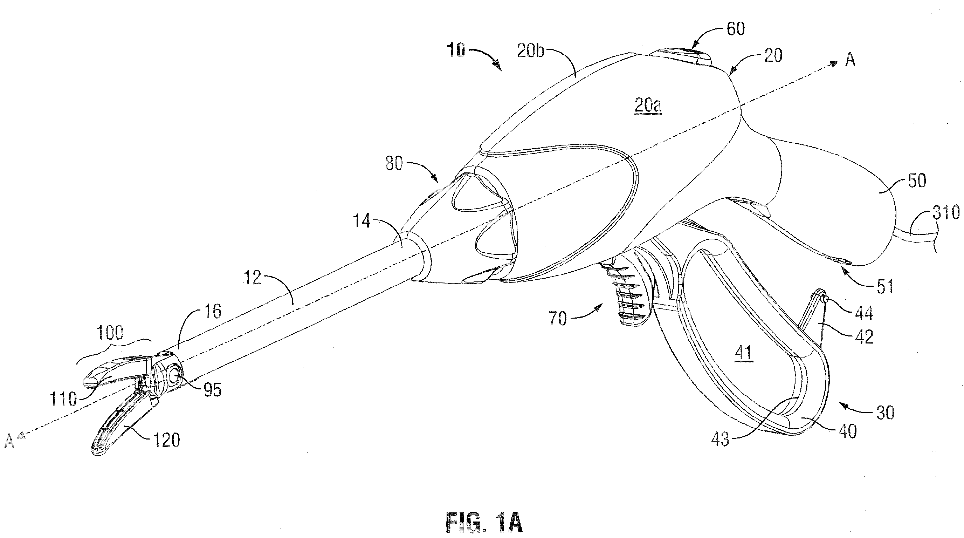

Figs. 1A-11 show in detail the operating features and inter-cooperating components of an endoscopic forceps for use with the present disclosure generally identified as forceps 10. For the purposes herein, forceps 10 is generally described.

Forceps 10 is for use with various surgical procedures and includes a housing 20, a handle assembly 30, a rotating assembly 80, a trigger assembly 70, a switch 60 and an end effector assembly 100 which mutually cooperate to grasp, seal and divide tubular vessels and vascular tissues. Forceps 10 includes a shaft 12 which has a distal end 16 dimensioned to mechanically engage the end effector assembly 100 and a proximal end 14 that mechanically engages the housing 20. Details of how the shaft 12 connects to the end effector 100 are described in more detail below. The proximal end 14 of shaft 12 is received within the housing 20 and the connections relating thereto are also described in detail below.

As best seen in Fig. 1A, forceps 10 also includes an electrosurgical cable 310 that connects the forceps 10 to an electrosurgical generator (not shown) such that upon activation of switch 60 energy is supplied to the end effector assembly 100 to energize tissue disposed therein.

Handle assembly 30 includes a fixed handle 50 and a movable handle 40. Fixed handle 50 is integrally associated with housing 20 and handle 40 is movable relative to fixed handle 50 as explained in more detail below with respect to the operation of the forceps 10. Rotating assembly 80 is operatively associated with the housing 20 and is rotatable approximately 180 degrees about a longitudinal axis "A-A" (See Fig. 1A).

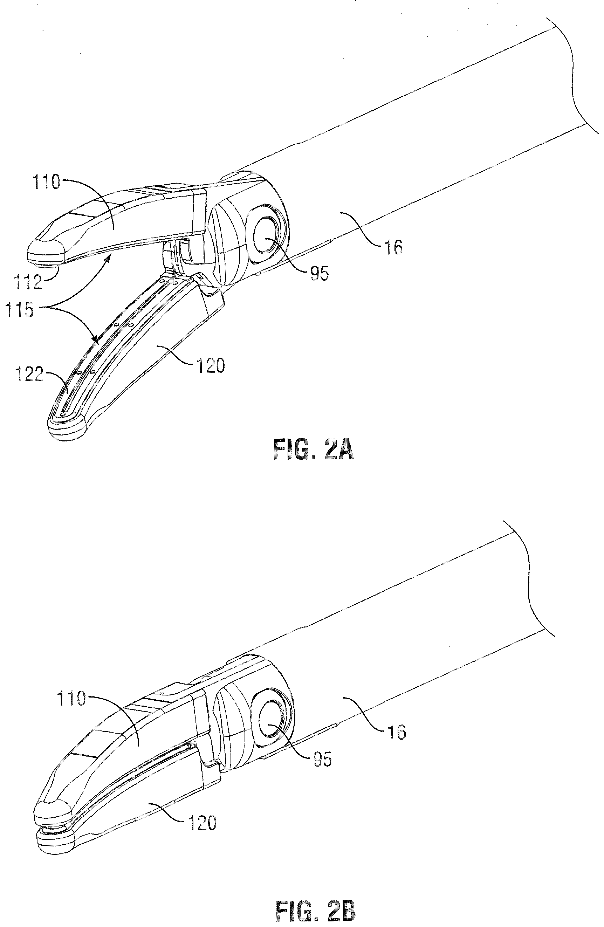

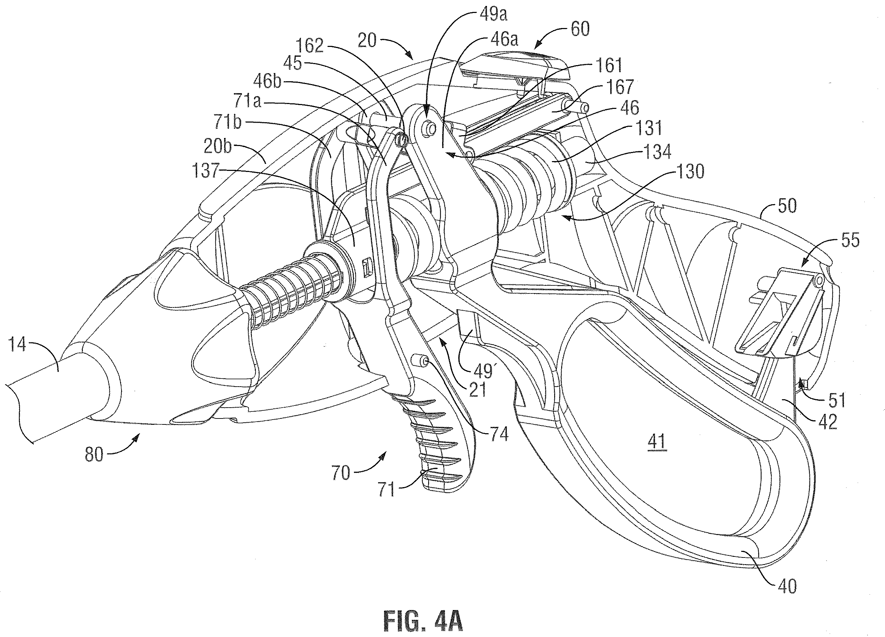

End effector assembly 100 is attached at the distal end 16 of shaft 12 and includes a pair of opposing jaw members 110 and 120. Movable handle 40 of handle assembly 30 is ultimately connected to a drive assembly 130 (See Fig. 4A) to impart movement of the jaw members 110 and 120 from an open position wherein the jaw members 110 and 120 are disposed in spaced relation relative to one another (See Fig. 2A), to a clamping or closed position wherein the jaw members 110 and 120 cooperate to grasp tissue therebetween (See Fig. 2B).

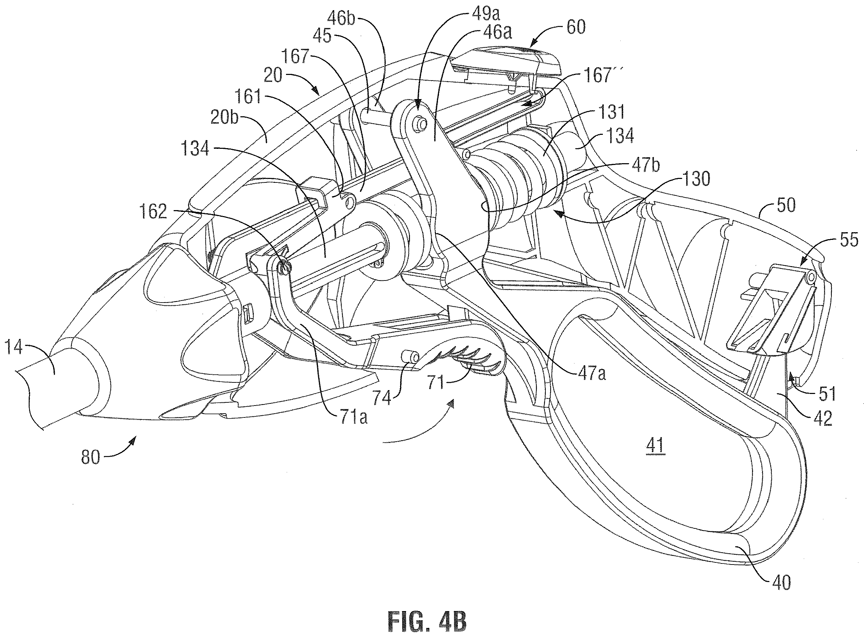

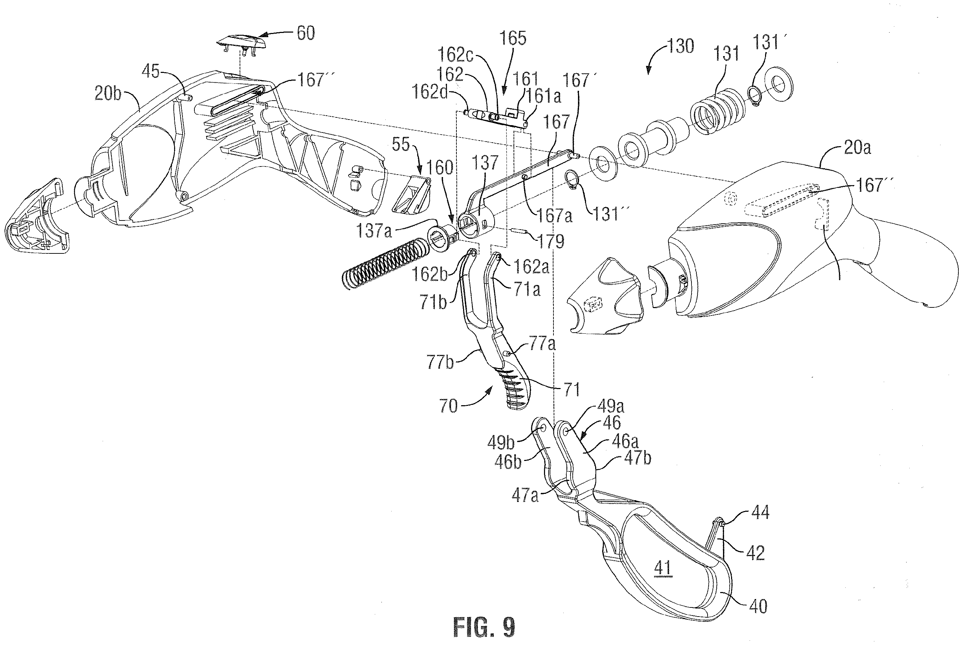

Movable handle 40 includes a finger loop 43 that has an aperture 41 defined therethrough that enables a user to grasp and move the handle 40 relative to the fixed handle 50. As best seen in Figs. 4A and 4B movable handle 40 is selectively movable about a pivot pin 45 from a first position relative to fixed handle 50 to a second position in closer proximity to the fixed handle 50 which, as explained below, imparts movement of the jaw members 110 and 120 relative to one another. The movable handle 40 includes a clevis 46 that forms a pair of upper flanges 46a and 46b each having an aperture 49a and 49b at an upper end thereof for receiving pivot 45 (See Fig. 9) therethrough and mounting the upper end of the handle 40 to the housing 20. In turn, pin 45 mounts to a respective housing half 20a and 20b.

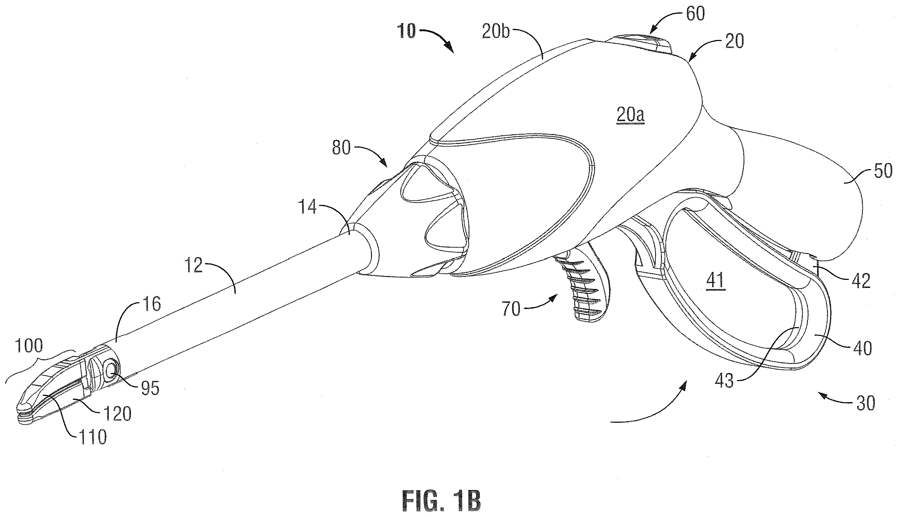

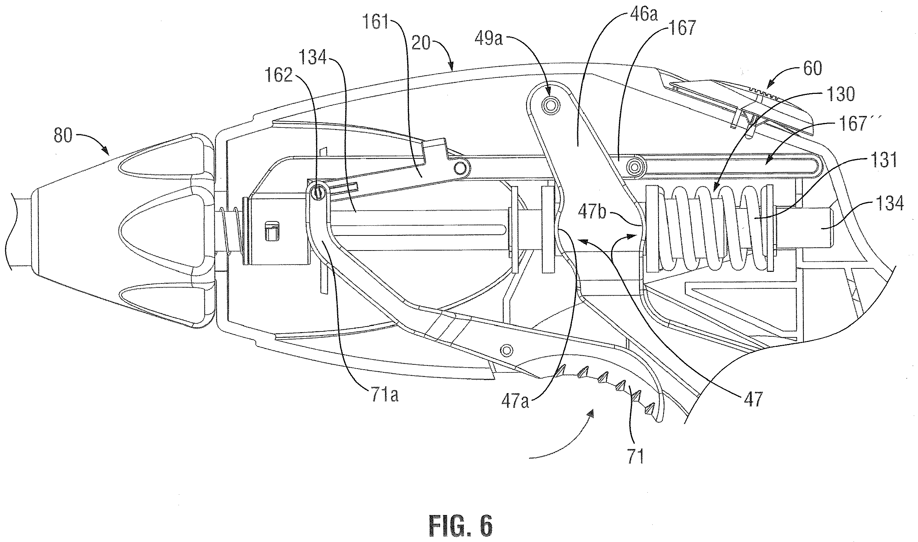

Each upper flange 46a and 46b also includes drive flanges 47a and 47b (collectively referred to as driving flange 47) (See Figs. 4B and 6), respectively, that are aligned along longitudinal axis "A-A" and which abut the drive assembly 130 such that pivotal movement of the handle 40 forces actuating flanges 47a and 47b against the drive assembly 130 which, in turn, closes the jaw members 110 and 120 (See Figs. 1A, 1B, 4A and 4B).

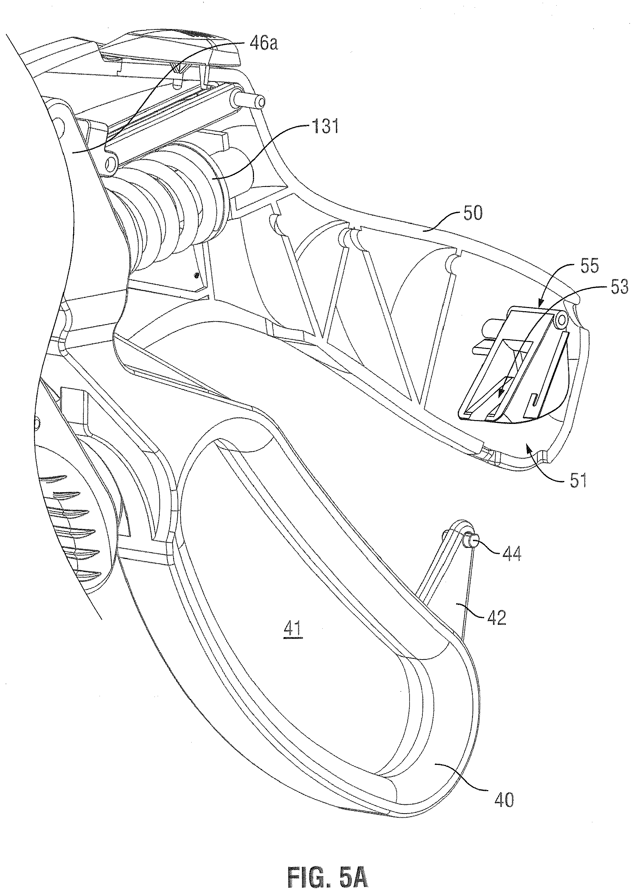

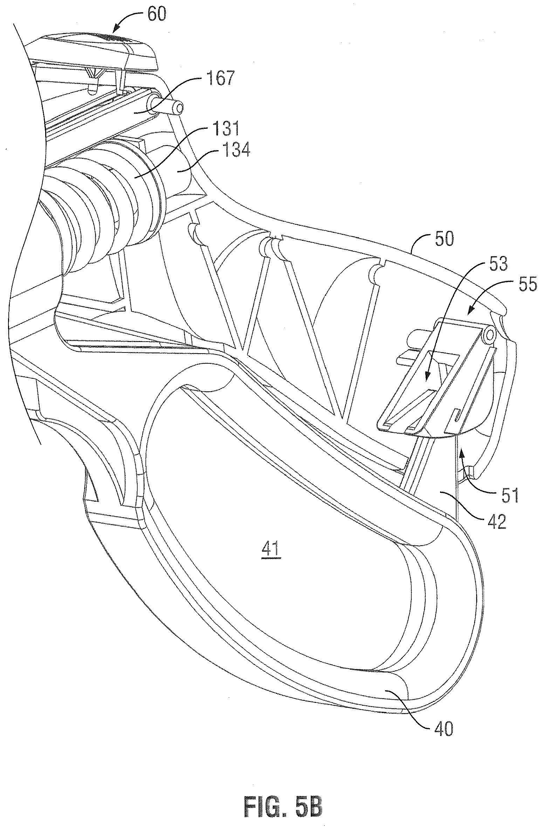

As shown in Figs. 5A and 5B, the lower end of the movable handle 40 includes a flange 42 that is operatively associated with movable handle 40. Flange 42 includes a t-shaped pin 44 that projects laterally or transversally from a distal end thereof that is configured to engage a corresponding railway 55 disposed within fixed handle 50. Pin 44 is configured to ride within a predefined channel 53 defined within the railway 55 to lock the movable handle 40 relative to the fixed handle 50 upon reciprocation thereof.

Movable handle provides a distinct mechanical advantage over conventional handle assemblies due to the unique position of the pivot pin 45 relative to the longitudinal axis "A-A" of the shaft 12 and the disposition of the driving flange 47 along longitudinal axis "A-A". In other words, by positioning the pivot pin 45 above the driving flange 47, the user gains mechanical advantage to actuate the jaw members 110 and 120 enabling the user to close the jaw members 110 and 120 with less force while still generating the required forces necessary to affect a proper and effective tissue seal.

As shown in Figs. 2A-3B, 10 and 11, the end effector assembly 100 includes opposing jaw members 110 and 120 that cooperate to effectively grasp tissue for sealing purposes. The end effector assembly 100 is designed as a bilateral assembly, i.e., both jaw members 110 and 120 pivot relative to one another about a pivot pin 95. The jaw members 110 and 120 are curved to facilitate manipulation of tissue and to provide better "line of sight" for accessing targeted tissues.

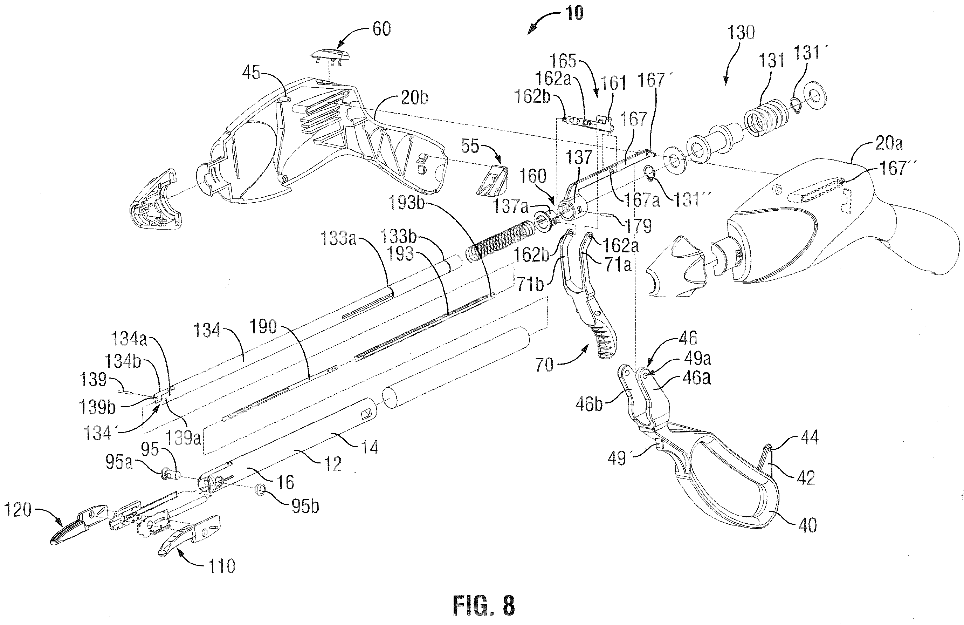

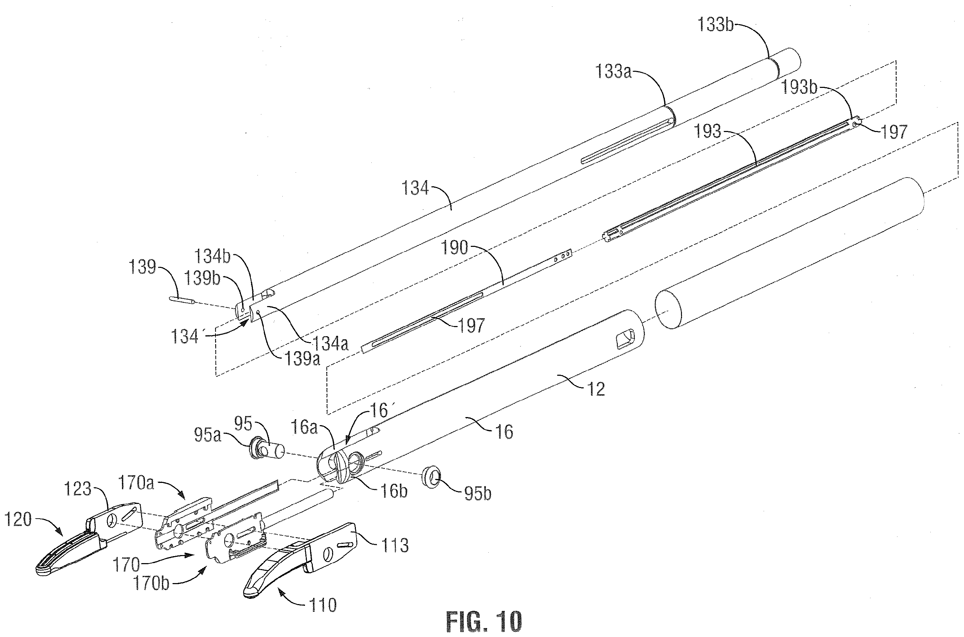

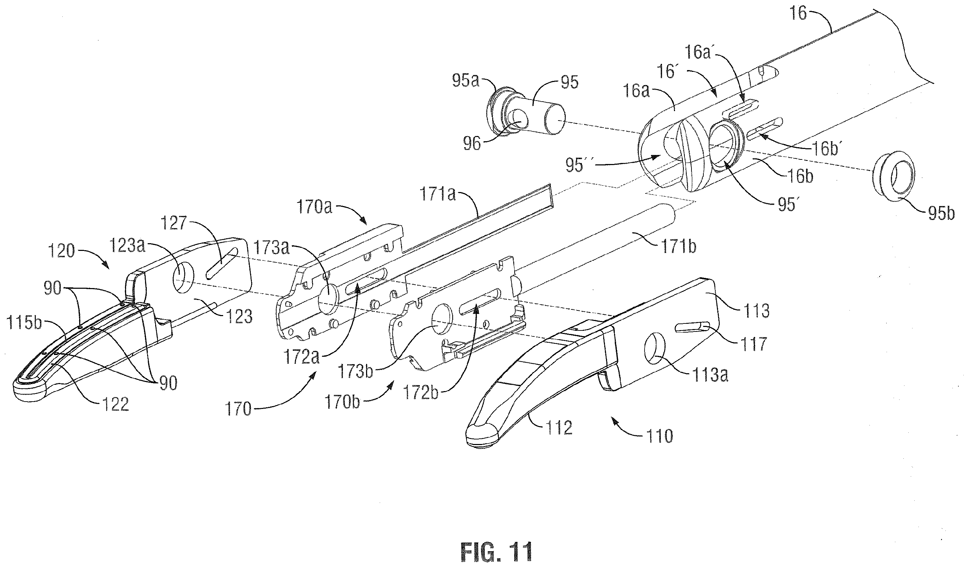

A reciprocating drive sleeve 134 (See Fig. 4A) is slidingly disposed within the shaft 12 and is remotely operable by the drive assembly 130. Drive sleeve 134 includes a bifurcated distal end composed of halves 134a and 134b (See Figs. 8 and 10), respectively, that define a cavity 134' therebetween for receiving jaw members 110 and 120. Jaw members 110 and 120 include proximal flanges 113 and 123, respectively, that each include an elongated angled slot 117 and 127, respectively, defined therethrough (See Fig. 11). A drive pin 139 (See Fig. 10) is slidingly engaged through grooves 117 and 127 of jaw members 110 and 120 and anchors the end of sleeve 134 within cavity 134' disposed between flanges 134a and 134b (See Fig. 10). Cam pin or drive pin 139 mounts through apertures 139a and 139b defined in flanges 134a and 134b, respectively and is reciprocable within slots 16a' and 16b' disposed at the distal ends 16a and 16b (See Figs. 10 and 11).

Drive sleeve 134, which ultimately connects to the drive assembly 130, slidingly receives knife drive rod 193, knife 190 and posts 171a and 171b of halves 170a and 170b of knife guide 170. Drive sleeve 134, in turn, is received within shaft 12. Upon actuation of the drive assembly 130, the drive sleeve 134 reciprocates which, in turn, causes the drive pin 139 to ride within slots 117 and 127 to open and close the jaw members 110 and 120 as desired. The jaw members 110 and 120, in turn, pivot about pivot pin 95 disposed through respective pivot holes 113a and 123a disposed within flanges 113 and 123. Squeezing handle 40 toward handle 50 pulls drive sleeve 134 and drive pin 139 proximally to close the jaw members 110 and 120 about tissue grasped therebetween and pushing the sleeve 134 distally opens the jaw members 110 and 120 for grasping purposes.

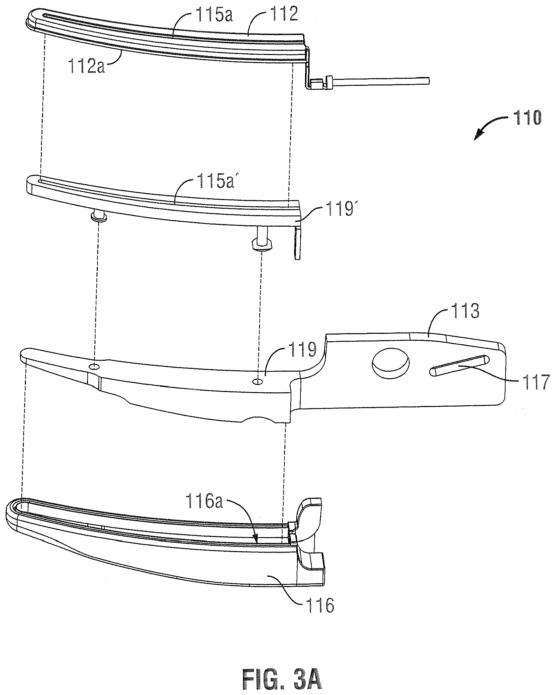

As shown in Fig. 3A, jaw member 110 includes a support base 119 that extends distally from flange 113 and that is dimensioned to support an insulative plate 119' thereon. Insulative plate 119' supports an electrically conductive sealing plate 112 thereon. Insulative plate 119' is affixed to support base 119 in an initial overmolding process. Support base 119 (together with the insulative plate 119') and electrically conductive sealing plate 112 are encapsulated by an outer insulative housing 116 added by way of a subsequent overmolding process. Outer housing 116 includes a cavity 116a that securely engages the electrically conductive sealing plate 112 as well as the support base 119 and insulative plate 119' during the overmolding process. The electrically conductive sealing plate 112 includes a seating or retaining flange 112a that securely seats sealing plate 112 within the housing during the overmolding process.

Sealing plate 112 and the outer housing 116, when assembled, form a longitudinally-oriented slot 115a defined therethrough for reciprocation of the knife blade 190 (See Fig. 10). Insulator plate 119' includes respective longitudinally-oriented knife slot 115a' defined therethrough for reciprocation of the knife blade 190. Knife slot 115a cooperates with a corresponding knife slot 115b defined in jaw member 120 to facilitate longitudinal extension of the knife blade 190 along a preferred cutting plane to effectively and accurately separate the tissue along the formed tissue seal. Together, knife slots 115a and 115b form knife channel 115 for reciprocation of the knife 190. As illustrated in Fig. 2A, knife channel 115 runs through the center of the jaw members 110 and 120, respectively, such that a blade 190 from the trigger assembly 70 can cut the tissue grasped between the jaw members 110 and 120 when the jaw members 110 and 120 are in a closed position. Handle 40 includes a passive lockout flange 49' that prevents actuation of the knife assembly 70 when the handle 40 is open (See Fig. 8).

End effector assembly 100 also includes knife guide 170 that facilitates alignment and translation of the knife 190 through and into the knife channel 115. Knife guide 170 includes half 170a and half 170b which mechanically interface to encapsulate the knife 190 upon assembly (See Figs. 10 and 11). Knife guide 170 aligns the knife 190 for facile translation through knife channel 115 upon reciprocation of a knife drive rod 193 (Fig. 10). Halves 170a and 170b include apertures 173a and 173b, respectively, defined therethrough that allow passage of the pivot 95 during assembly (See Fig. 11). Halves 170a and 170b also include laterally-aligned slots 172a and 172b defined therein that allow reciprocation of the drive pin 139 upon opening and closing of the jaw members 110 and 120. Knife guide halves 170a and 170b also include posts 171a and 171b that extend proximally into slots 16' and 134', respectively, upon assembly to engage knife 190 (See Figs. 10 and 11).

The knife 190 can only be advanced through the tissue when handle 40 is closed thus preventing accidental or premature activation of the knife 190 through the tissue. As mentioned above, passive lockout flange 49' prevents unintended translation of the knife 190 while the jaw members 110 and 120 are disposed in an open configuration.

Jaw member 120 includes similar elements to jaw member 110 such as jaw housing 126 which encapsulates a support plate 129, an insulator plate 129' and sealing plate 122. Likewise, the electrically conductive surface or sealing plate 122 and the insulator plate 129' include respective longitudinally-oriented knife slots 115b and 115b' defined therethrough for reciprocation of the knife blade 190. When the jaw members 110 and 120 are closed about tissue, knife slots 115a and 115b form a complete knife channel 115 to allow longitudinal extension of the knife 190 in a distal fashion to sever tissue along a tissue seal. Jaw member 120 is assembled in a similar manner as described above with respect to jaw member 110.

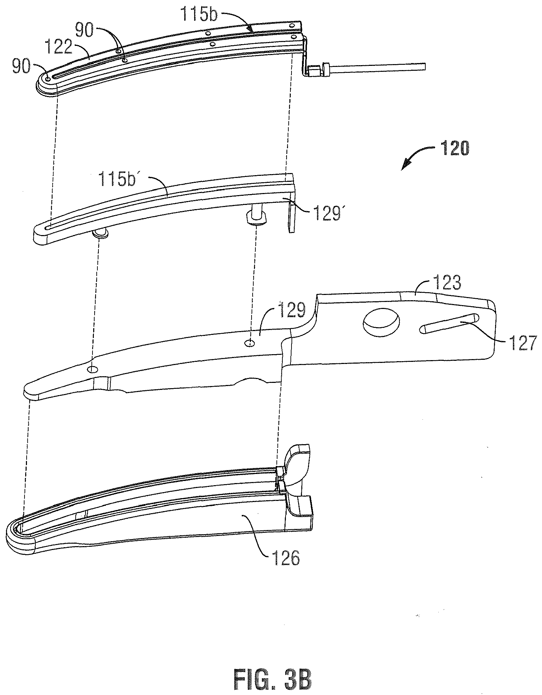

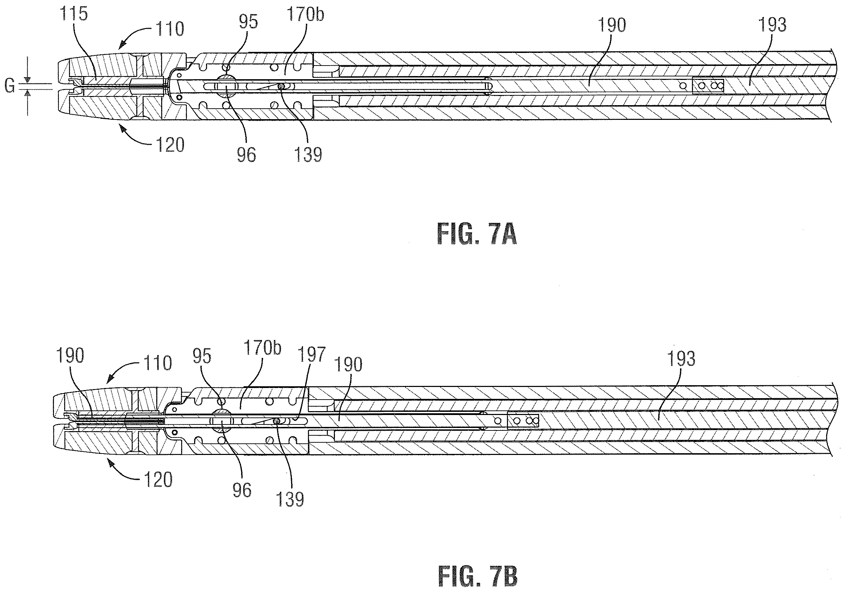

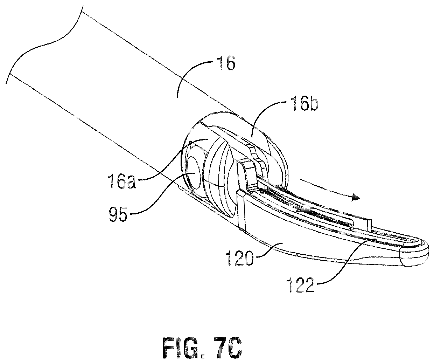

As seen in Fig. 3B, jaw member 120 includes a series of stop members 90 disposed on the inner facing surface of the electrically conductive sealing surface 122 to facilitate gripping and manipulation of tissue and to define a gap "G" (Fig. 7A) between opposing jaw members 110 and 120 during sealing and cutting of tissue. The series of stop members 90 are applied onto sealing plate 122 during manufacturing.

Jaw members 110 and 120 are electrically isolated from one another such that electrosurgical energy can be effectively transferred through the tissue to form a tissue seal. The two electrical potentials are isolated from one another by virtue of the insulative sheathing surrounding the conductive leads.

Jaw members 110 and 120 are engaged to the end of rotating shaft 12 by pivot pin 95 such that rotation of the rotating assembly 80 correspondingly rotates shaft 12 (along with sleeve 134 and knife 190) which, in turn, rotates end effector assembly 100 (See Fig. 1A). The distal end of rotating shaft 12 is bifurcated to include ends 16a and 16b that define a channel 16' therein for receiving jaw members 110 and 120. Pivot pin 95 includes a stem 95a and cap 95b arrangement which is dimensioned to engage through aperture 95' and 95" disposed in ends 16b and 16a, respectively (See Fig. 11). Pivot 95 is sizeable to provide adequate pivot strength and also to include an aperture 96 disposed therethrough which allows reciprocation of knife 190.

Upon assembly as illustrated in Figs. 10 and 11, the stem 95a of pivot pin 95 extends through end 16a of shaft 12, aperture 123a of jaw member 120, aperture 173a of half 170a of knife guide 170, aperture 173b of half 170b of knife guide 170, aperture 113a of jaw member 110 and end 16b of shaft 12 to engage cap 95b. Slots 16a' and 16b' are defined within distal ends 16a and 16b, respectively, and allow reciprocation of drive pin 139.

Figs. 4A, 4B, 8 and 9 show the details of the housing 20 and the component features thereof, namely, the drive assembly 130, the rotating assembly 80, the knife actuating assembly 160, the trigger assembly 70 and the handles 40 and 50. Figs. 4A and 4B show the above-identified assemblies and components in an assembled form in the housing 20 and Figs. 8 and 9 show an exploded view of each of the above-identified assemblies and components.

Actuation of the handle 40 along with the inter-cooperating elements of the drive assembly 130 cooperate to close the jaw members 110 and 120 about tissue with a predeterminable and consistent closure pressure to affect a tissue seal. As mentioned above, closure pressures for sealing large tissue structures fall within the range of about 3kg/cm² to about 16 kg/cm².

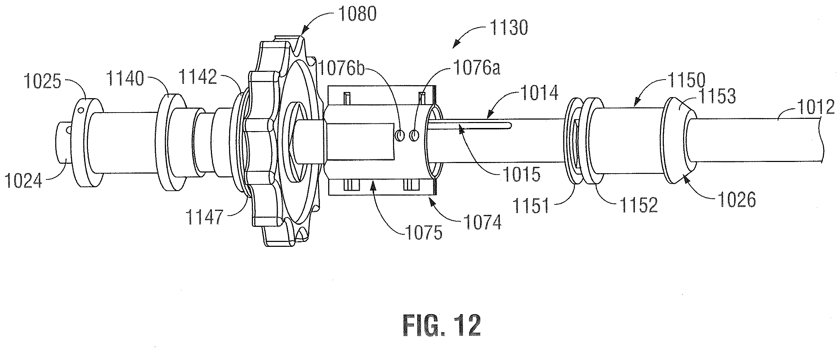

Figs. 8 and 9 show forceps 10 that is assembled during the manufacturing process. As can be appreciated, many components are arranged, sequenced and assembled to construct the forceps 10. For example, the drive assembly 130 mounts atop the proximal portion of the drive sleeve 134. A pair of retaining rings 131' and 131" cooperate with a corresponding pair of relieved portions 133a and 133b disposed on the drive sleeve 134 to mount the drive assembly 130 atop the drive sleeve 134 such that relative movement of the drive assembly 130 correspondingly moves the drive sleeve 134. As handle 40 pivots about pivot point 45 and moves relative to handle 50 and flange 42 is incorporated into channel 51 of fixed handle 50, the driving flanges 47a and 47b, through the mechanical advantage of the above-the-center pivot point, force the drive assembly 130 proximally against spring 131. As a result thereof, drive sleeve 134 reciprocates proximally which, in turn, closes the jaw members 110 and 120. As a result, a predeterminable closure force is transmitted to the opposing jaw members 110 and 120. As discussed below with reference to Figs. 12 -17, a modular design simplifies the illustrated assembly process and allows the manufacturer to quickly and easily assemble a range of different forceps with different shaft designs for different surgical needs.

Figs. 4A, 4B and 9 show a trigger assembly 70 for use with a prior forceps. More particularly, trigger assembly mounts atop movable handle 40 and cooperates with the knife assembly 160 to selectively translate knife 190 through a tissue seal. The trigger assembly 70 includes a U-shaped finger actuator 71 having a pair upwardly-extending flanges 71a and 71b (See Fig. 9). Flanges 71a and 71b include apertures 162a and 162b that engage a corresponding pair of detents 162c and 162d located on knife carriage 165. Finger actuator 71 is selectively pivotable within a predefined slot 21 (See Fig. 4A) disposed within housing 20. A pivot pin having a pair of projections or pivots 77a and 77b disposed on either side of the finger actuator 71 mounts the finger actuator 71 between housing halves 20a and 20b to pivot the finger actuator 71 within slot 21.

The knife assembly 160 includes a reciprocating knife bar 167 that mounts atop the drive sleeve 134 and between upwardly extending flanges 71a and 71b. Knife bar 167 includes a t-shaped proximal end 167' and a cuff 137 disposed at the distal end thereof. Cuff 137 is dimensioned to encapsulate drive sleeve 134 when the knife assembly 160 is assembled. Proximal end 167' is dimensioned to mount and slidingly reciprocate within a slot 167" formed by housings 20a and 20b at assembly (See Fig. 9). A locking cap 137a and a mounting pin 179 secure the cuff 137 to the proximal end 193b of the knife rod 193 through aperture 197 disposed therein such that proximal movement to the finger actuator 71 results in distal movement of the knife bar 193 (See Figs. 9 and 10). Knife carriage 165 mounts to the upwardly extending flanges 71a and 71b of the finger actuator 71. The distal end 162 of the knife carriage 165 is t-shaped and includes two laterally extending pins 162c and 162d which engage apertures 162a and 162b, respectively, in flanges 71a and 71b. The proximal end 161 of the knife carriage 165 includes an aperture 161a defined therein which mates with a detent 167a that extends transversally through knife carriage 165. Again, and as discussed below with reference to Figs. 12 -17, a modular design of the shaft and drive assembly simplifies the trigger assembly 70 and knife assembly 160 and allows the manufacturer to quickly and easily assemble a range of different forceps with easily configurable trigger assemblies and stroke lengths.

As illustrated in Fig. 4A, when the handle 40 is disposed in a spaced-apart or open configuration relative to handle 50, flange 49' that extends from handle 40 prevents actuation of the trigger assembly 70. Finger actuator 71 is prevented from being actuated proximally by flange 49' when the jaw members 110 and 120 are open. This prevents premature actuation of the knife 190 when tissue is not grasped between jaw members 110 and 120. When handle 40 is selectively moved relative to handle 50, gap 21 is formed between the flange 49' and the finger actuator 71 (See Fig. 4A). The user is free to selectively actuate the knife 190 by squeezing the finger actuator 71 proximally within gap 21.

As shown in Figs. 4B, 6A and 6A, once the clearance is provided by movement of handle 40, proximal movement of the finger actuator 71 about pivot 74 results in distal translation of the knife bar 167. This in turn, results in distal translation of the knife rod 193 and knife 190. When finger actuator 71 is squeezed proximally, the U-shaped flanges 71a and 71b rotate about pivot 74 to abut cuff 137 and essentially throw the knife carriage 165 forward which, in turn, carries the knife bar 167 forward to force the knife rod 193 distally. As shown in Figs. 7A and 7B, distal translation of the knife rod 193 translates the knife 190 through aperture 96 in pivot 95 and through channel 115 in the jaw members 110 and 120. A slot 197 defined within the knife 190 provides clearance for pin 139 of the drive sleeve 134 during reciprocation of the knife 190.

Switch 60 is ergonomically dimensioned and conforms to the outer shape of housing 20 (See Figs. 1A and 1B). Switch 60 is designed to allow a user to selectively activate the jaw members 110 and 120. When switch 60 is depressed, a voltage drop is relayed to and recognized by the generator (not shown) to initiate electrical activation of the jaw members 110 and 120. Switch 60 acts as a control circuit and is protected or removed from the actual current loop which supplies electrical energy to the jaw members 110 and 120. This reduces the chances of electrical failure of the switch 60 due to high current loads during activation.

After the tissue is grasped between jaw members 110 and 120, the forceps 10 is ready for selective application of electrosurgical energy and subsequent separation of the tissue. By controlling the intensity, frequency and duration of the electrosurgical energy and pressure applied to the tissue, the user can effectively seal tissue.

Once a tissue seal forms isolating two tissue halves, the knife assembly 160 when activated via the trigger assembly 70, progressively and selectively divides the tissue along an ideal tissue plane in a precise manner to effectively and reliably divide the tissue into two sealed halves.

Figs. 12-17 show embodiments of modular shaft assemblies for use with the presently-described forceps 10 or other forceps described herein. Obviously, certain features of the forceps 10 may need to be modified to incorporate the modular shaft assemblies and certain of these features are explained in detail below. As can be appreciated, the purpose behind the modular shaft assembly is to construct a single or common shaft assembly that may be utilized for a variety of different instruments with little or no modification to the shaft during assembly. As explained below, certain features of the shaft assembly may be easily positioned, adjusted or modified (e.g., the knife assembly) to fit or accommodate a variety of different forceps designs.

Fig. 12 shows one embodiment of a modular shaft assembly 1130 that includes proximal and distal ends 1024 and 1026 respectively. A shaft 1012 is mechanically coupled to the distal end thereof (as explained in more detail below) and the proximal end 1024 is configured for selective engagement within a forceps housing, e.g., housing 20, via a proximal spindle 1025. A pair of drive rings 1140 and 1142 are biased atop the proximal end 1024 of the shaft assembly 1130 and are configured to slide thereon relative to one another upon actuation of the handle 40 in a similar manner as described above. Squeezing handle 40 toward handle 50 pulls drive sleeve and drive pin proximally (not shown - but see drive sleeve 134 and drive pin 139) to close the jaw members 110 and 120 about tissue grasped therebetween and pushing the sleeve distally opens the jaw members 110 and 120 for grasping purposes (See Fig. 6). A compression spring 131 (See Fig. 4A) is employed between the proximal spindle 1025 and the proximal drive ring 1140 and once the jaw members 110 and 120 close about tissue, the drive assembly 1130 bottoms out (i.e., further proximal movement of the reciprocating drive sleeve is prevented) and further movement of handle 40 compresses spring 131 resulting in additional closure force on the tissue. Spring 131 also tends to bias the jaw members 110 and 120 and the movable handle 40 in an open configuration.

A rotating assembly 1080 is affixed for rotation atop the shaft assembly 1130 and operates in a similar manner as described above. The relative position of the rotation assembly 1080 may be fixed and the various forceps may be configured to accommodate a fixed rotation assembly or the rotating assembly may be manufactured with some degree of play to accommodate for varying forceps designs.

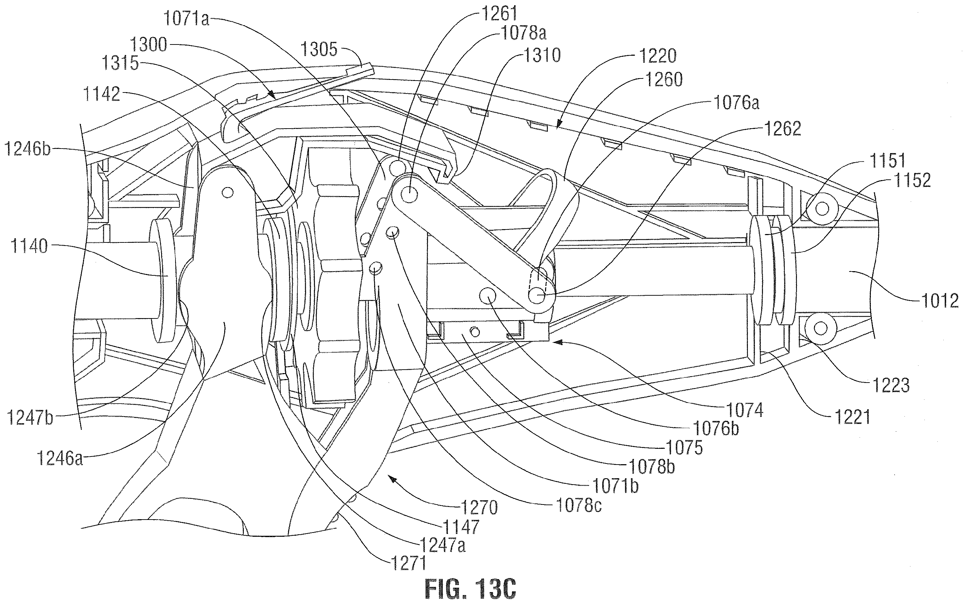

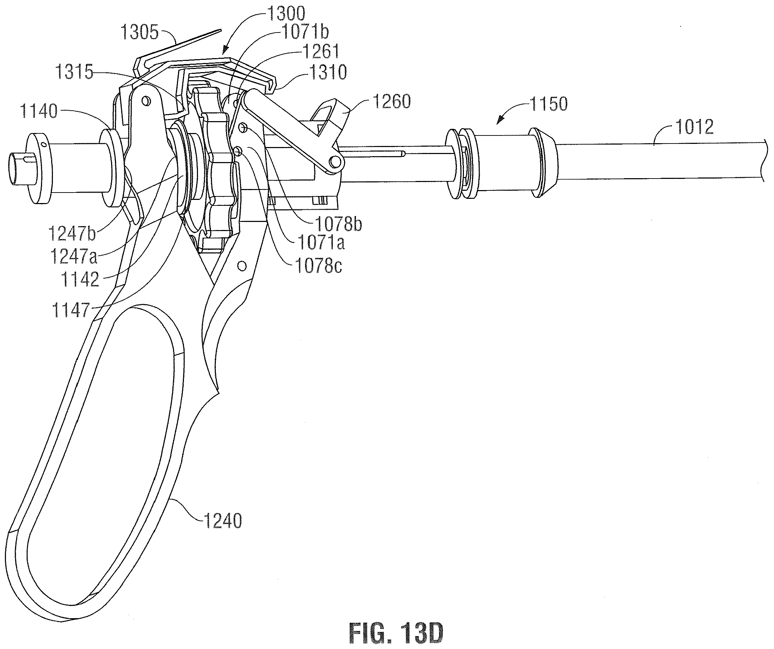

A knife assembly 1074 is also affixed atop a midway portion 1014 of the shaft assembly 1130 and includes a knife actuator 1075 that slideably mounts atop the midway portion 1014 of the shaft assembly 1130. An elongated slot 1015 is defined within the midway portion 1014 of the shaft assembly 1130 and cooperates with the knife actuator 1075 to advance and retract the knife rod 193 in a similar manner as described above with respect to Figs. 8 and 10. As explained in more detail below with reference to the remaining figures, the knife actuator 1075 may be selectively positioned or adjusted according to the required stroke length of the knife 190 for different forceps. In this instance the knife actuator 1075 may include a series of apertures 1076a and 1076b disposed therealong that engage pin 1262 (See Fig. 13C) to vary the position of the knife actuator 1075.

The distal end 1026 of the shaft assembly 1130 includes a tapered bushing 1150 mounted thereon. The outer diameter and shape of the bushing 1150 is uniform for the shaft assembly 1130 to facilitate assembly into various forceps designs but the inner dimensions may vary depending upon the size of the outer diameter of the shaft 1012. An interchangeable bushing 1150 may also be utilized to accommodate the various shaft diameters or the inner periphery may include any known type of mechanical coupling to accommodate shaft sizes, bayonet, slide-fit, snap-fit, screw-fit, etc.

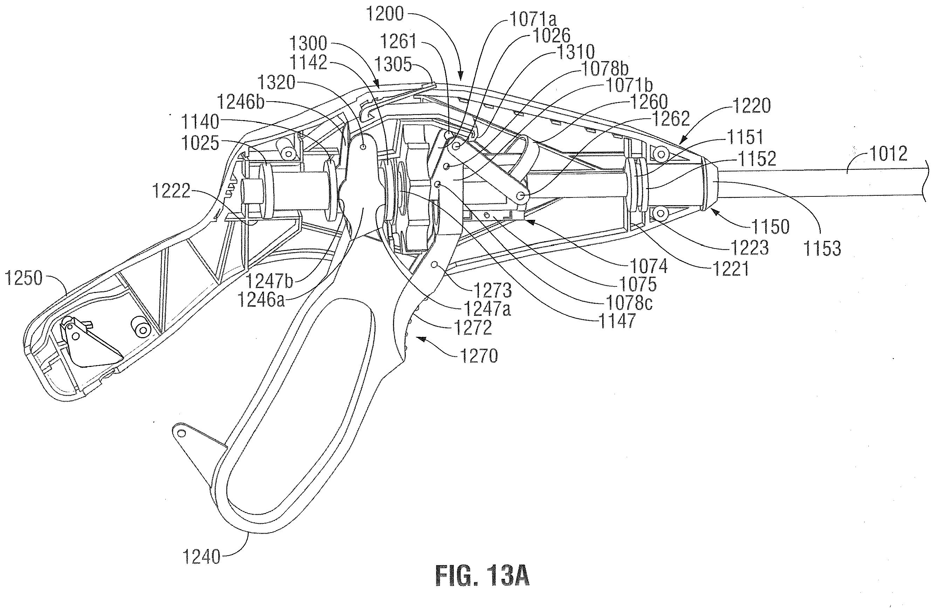

As best shown in Fig. 13A, to facilitate assembly of the shaft assembly 1130 within an internal forceps housing 1220, the bushing 1150 of the shaft assembly 1130 includes a series of mechanical rings 1151 and 1152 that cooperatively engage a series of corresponding grooves 1221 and 1223 to mount and secure the shaft assembly 1130 within the forceps housing 1220. The distal end 1153 of the bushing 1150 is also tapered to secure the shaft assembly 1130 within the forceps housing 1220.

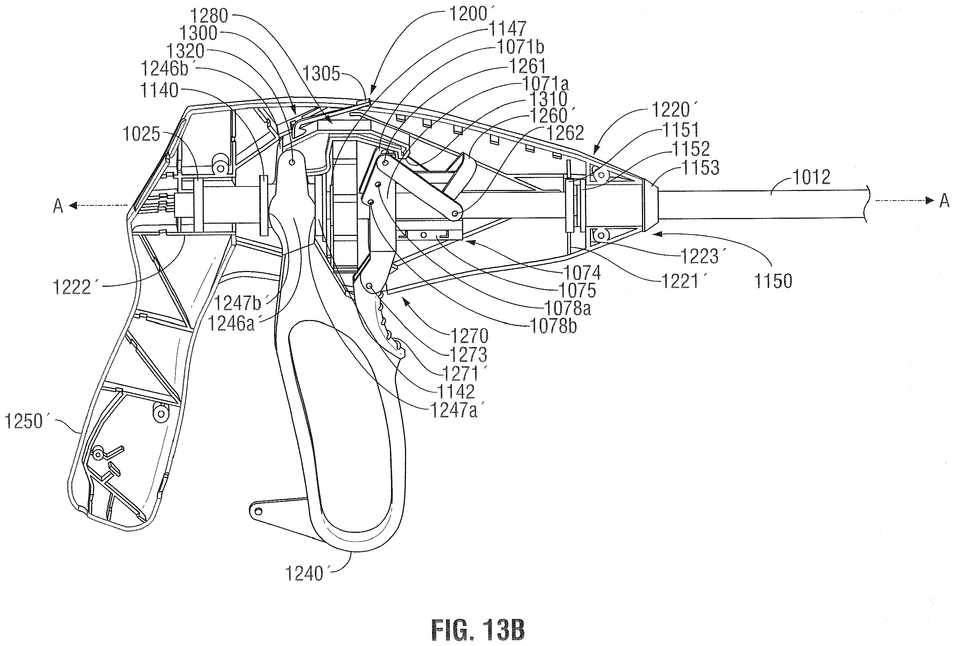

Figs. 13A and 13B show the same shaft assembly 1130 mounted into two different forceps housings 1220 and 1220', respectively. More particularly, Fig. 13A shows a forceps 1200 having an approximate thirty degree (30°) handle design relative to axis A-A and Fig. 13B shows a forceps 1200' having an approximate ninety degree (90°) handle design relative to axis A-A. As can be appreciated, the two different forceps 1200 and 1200' include different designs but uniform internal mechanical interfaces (e.g., grooves 1221 and 1223 and rear shelf support 1222) that cooperatively engage corresponding mechanical interfaces on the shaft assembly 1130 (e.g., rings 1151, 1152 and proximal spindle 1025). Moreover, each forceps 1200 and 1200' includes common elements that connect to the above-mentioned various parts of the shaft assembly 1130. More particularly, forceps 1200 includes a handle 1240 that is selectively moveable relative to a fixed handle 1250 that extends from the longitudinal axis A-A at an angle of about thirty degrees (30°).

Movable handle 1240 includes a pair of upper flanges 1246a and 1246b each having a pair of drive flanges 1247a and 1247b, respectively, that are aligned along longitudinal axis "A-A" and that correspondingly abut respective drive rings 1142 and 1140 of the shaft assembly such that pivotal movement of the handle 1240 forces actuating flanges 1247b and 1247b against the respective drive rings 1142 and 1140 which, in turn, closes the jaw members 110 and 120 about tissue.

Forceps 1200 also includes a trigger assembly 1270 that operably couples to the knife actuator 1075 such that movement thereof actuates the knife 190 disposed between the jaw members 110 and 120. More particularly, the trigger assembly 1270 includes a finger actuator 1272 and a pair of upwardly extending flanges 1071a and 1071b that operably engage (on either sides thereof) the knife actuator 1075 via a knife stroke link 1260. A pair of adjustment pins 1261 (only one pin shown) couples the knife stroke link 1260 at a proximal end thereof to each upwardly extending flange 1071a and 1071b, respectively. A second pin 1262 couples the knife stroke link 1260 to the distal end of the knife actuator 1075.

It is contemplated that each upwardly extending flange, e.g., 1071a, may include a series of apertures 1078a, 1078b and 1078c disposed therein that mechanically engage pin 1261 at various positions along each upwardly extending flange 1071a such that the relative distance of the knife stroke link 1260 to the distal end of the knife actuator 1075 may be adjusted to vary the overall length of the knife stroke. In other words, the knife stroke length may be easily varied depending on the type of shaft 1012 attached to the distal end of the forceps housing 1220 that may include jaw members of varying length. It is also envisioned that the knife stroke link 1260 may include a series of apertures 1078a-1078c (or the knife actuator 1075 may include a series of apertures 1076a and 1076b) along a length thereof that operate in a similar manner to adjust the overall knife stroke length. In this instance the knife actuator 1075 acts as a second adjustable link.

During manufacture and assembly, once an assembly technician determines the type of shaft 1012 (e.g., size, jaw length, knife stroke length), the assembly technician can easily adjust the knife stroke length by adjusting the position of the pin 1261 within a specified aperture, e.g., 1078a, along the upwardly extending flanges, e.g., 1071a. A series of indications, graduations, or a color-coded system may be implemented to facilitate the assembly process. For example, the shaft 1012 may include a particular color, e.g., green, that signals the assembly technician to adjust the knife stroke link 1260 to the aperture, e.g., aperture 1078b, marked with the corresponding green color. A number or letter system may also be utilized for the same purpose.

Fig. 13A also shows one example of a knife lockout 1300 that operates as a safety mechanism to prevent the knife 190 from advancing when the jaw members 110 and 120 are disposed in an open configuration. As mentioned above, handle 1240 may include a passive lockout flange (not shown) that prevents actuation of the knife 190 when the handle 1240 is open.

With respect to Figs. 13A, knife lockout 1300 prevents actuation of the knife 190 until the handle 1240 is fully closed about tissue. More particularly, knife lockout 1300 operably couples in a pivotable fashion between upper flanges 1246a and 1246b and includes a hook-like distal end 1310 and a living hinge or spring 1305 that biases the knife lockout 1300 in an engaged position. A pivot 1320 connects the knife lockout 1300 to the upper flanges 1246a and 1246b. When disposed in an engaged position (e.g., when handle 1240 is disposed in an open configuration relative to the handle 1250), the hook-like distal end 1310 of the lockout 1300 is configured to operably engage pin 1261 of the trigger assembly 1270 thereby preventing actuation of the trigger assembly 1270 to advance the knife 190. A lower cam surface 1315 of the knife lockout 1300 is configured to engage a drive washer 1147 disposed adjacent the drive ring 1142 of the shaft assembly 1130 when the handle 1240 is disposed in an open configuration.

As best seen in Fig. 13C, when the handle 1240 is actuated to close the jaw members 110 and 120 about tissue, rotation of the upper flanges 1246a and 1246b to a fully actuated position causes drive washer 1147 to disengage from cam surface 1315 which, in turn, allows the distal end 1310 of the knife lockout 1300 to pivot and disengage from the pin 1261 thereby releasing the knife actuator 1075 for actuation via trigger assembly 1270. When the handle 1240 is released, the drive washer 1147 is moved proximally (against the force of spring 1305) to cause the hook-like distal end 1310 to re-engage pin 1261 and prevent translation of the knife 190 (or actuation of the trigger assembly 1270).

As mentioned above, Fig. 13B shows the same modular shaft assembly 1130 disposed within a different forceps 1200' having a different handle design, e.g., ninety-degree handle design). Forceps 1200' includes the same uniform elements or mechanical interfaces to mount the shaft assembly 1130 therein, e.g., 1221', 1223', 1222'. In addition, the forceps 1200' includes similar elements that cooperate to actuate the jaw members 110 and 120 and advance the knife 190 therebetween, e.g., handles 1240' and 1250', upper flanges 1246a' and 1246b', trigger assembly 1270' (and working components thereof - finger actuator 1271', upwardly extending flanges 1071a' and 1071b' with apertures 1078a'-1078c'), knife lockout 1300' (and working components thereof - hook-like distal end 1310', cam surface 1315' and spring 1305'), and knife stroke link 1260' (and working components thereof- pins 1261' and 1262'). All of these components cooperate in a similar fashion as described above with respect to forceps 1200 of Fig. 13A and are shown to illustrate the versatility of the modular shaft assembly 1130.

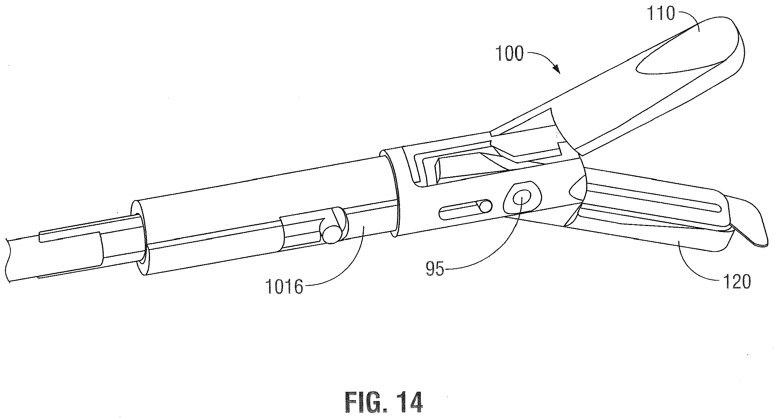

Fig. 14 shows a distal end of the shaft assembly 1130 that is configured to operably engage the end effector assembly 100 with the pair of opposing jaw members 110 and 120. In one embodiment, the shaft assembly 1130 may be configured to include a modularized distal tip 1016 wherein any number of differently-sized and configured end effector assemblies and jaw arrangements may be selectively engaged with the same distal end 1016 and same components of the shaft assembly 1130 for actuating the jaw members 110 and 120 and actuating the knife 190 as described above. For example, in one instance and to suit a particular surgical purpose, a unilateral end effector assembly may be selectively engaged with the distal end 1016 and in another instance a bilateral end effector assembly may be utilized. Various known coupling mechanisms (not shown) may be employed for this purpose.

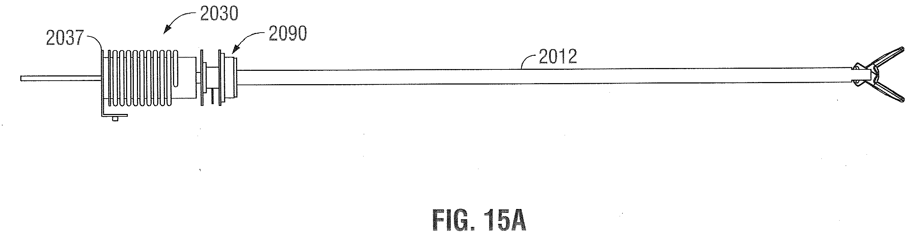

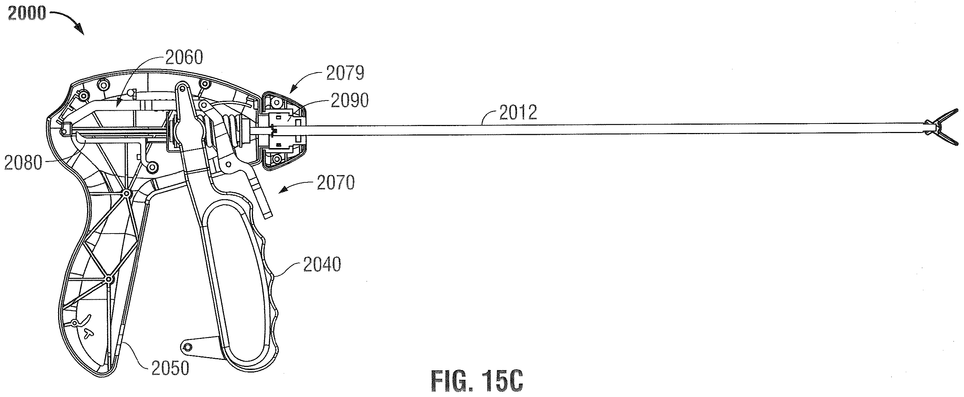

Figs. 15A-15D show another envisioned embodiment of a modular shaft assembly 2030 that may be utilized with a variety of different forceps, e.g., forceps 2000 (See Fig. 15C), having a variety of different shaft configurations 2012 and 2012'. Shaft assembly 2030 includes many of the same components as mentioned above with respect to shaft assembly 1030 and operates in a similar fashion, e.g., the shaft assembly 2030 maybe utilized with various forceps designs and with various shaft configurations.

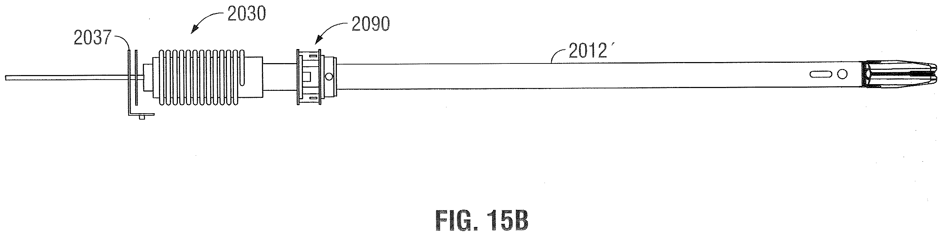

As best shown in Figs. 15A and 15B, shaft assembly 2030 includes a universal bushing or coupling element 2090 that is utilized to engage shafts 2012 and 2012' of varying sizes for use with different surgical purposes. For example, shaft 2012 is a 5mm x 37cm shaft for use with a 5mm port or trocar and shaft 2012' is a 10mm x 22cm shaft for use with a 10mm port or trocar. Other shaft configurations are also contemplated and may be interchanged at assembly or prior to use depending upon a particular surgical purpose. Similar to the bushing 1150, bushing 2090 is configured to mount within many different forceps of varying shape, size and dimension.

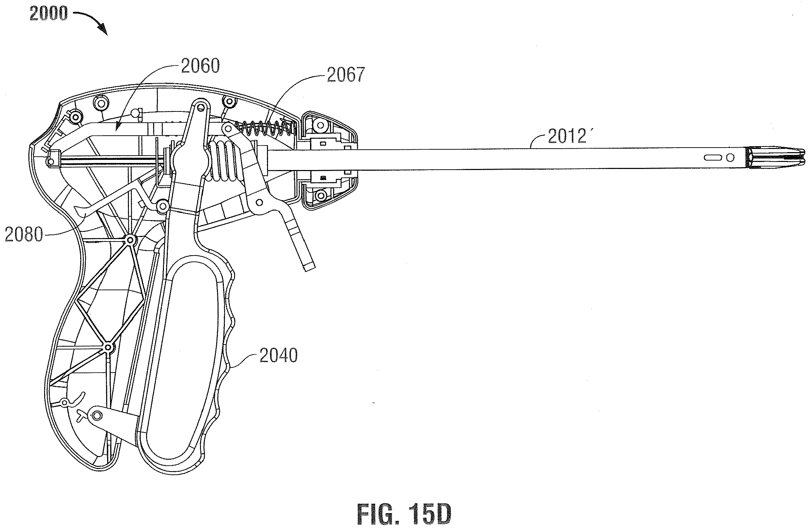

Figs. 15C and 15D show both shafts 2012 and 2012' mounted within the same forceps 2000. Forceps 2000 is similar to the forceps described above and includes fixed handle 2050 and movable handle 2040, rotating assembly 2079 and trigger assembly 2070 that all cooperate to grasp, seal and sever tissue. During manufacture or prior to assembly, a shaft size is determined and simply engaged to the distal end of the forceps 2000 to suit a particular surgical purpose. If the shaft 2012 is assembled to the forceps during a manufacturing step, other components, e.g., rotating assembly 2079 can be easily assembled in a subsequent assembly step, and the bushing 2090 may be manually attached to the shaft. In this instance, when the shaft 2012 is assembled by a user prior to use, a more simplified and automatically-engaging bushing 2090 may prove more useful, e.g., snap-fit or luer-like bushing-to-shaft interface. In either instance, the internal operating and actuating components of the shaft, e.g., shaft 2012, and the shaft assembly 2030 align and operably engage one another such that the forceps 2000 can operate as intended.

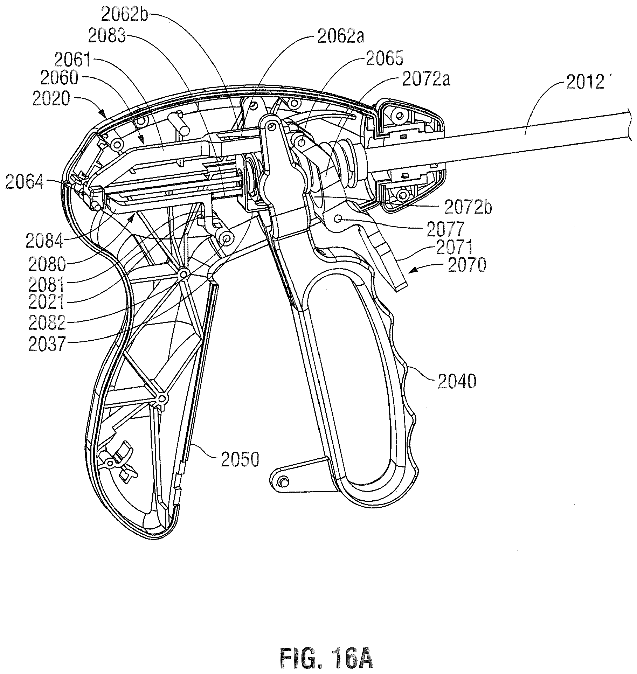

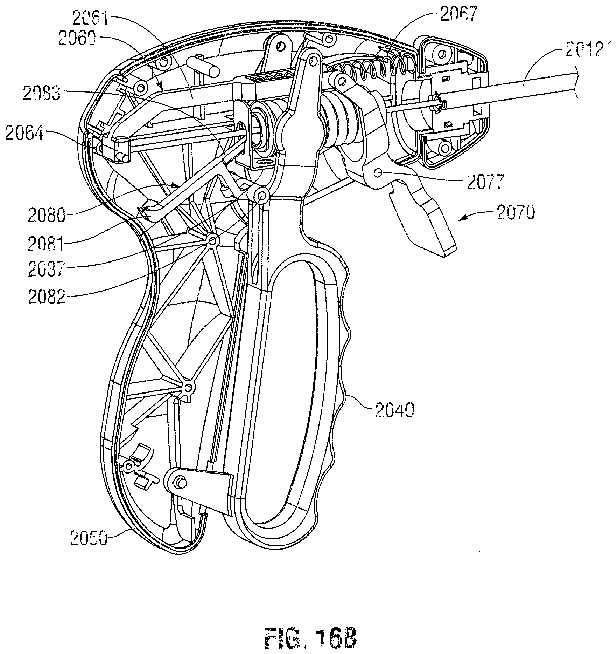

Figs. 15C, 15D and 16A and 16B show another embodiment of a knife lockout assembly 2060 for use with the forceps 2000 to prevent accidental advancement of the knife 190 when the jaw members 110 and 120 are disposed in an open configuration. Knife lockout assembly 2060 operates in much the same manner as the above-described knife lockout 1300 in that the jaw members 110 and 120 must be fully closed about tissue before the trigger assembly 2070 (and hence the knife 190) may be selectively advanced to cut tissue disposed between the jaw members 110 and 120. More particularly and in this instance, two knife safety mechanisms are employed: 1) a passive safety as described above wherein the movable handle 2040 blocks the trigger assembly 2070 from actuating in a proximal direction until the handle 2040 is squeezed closer to handle 2050; and 2) a knife lockout assembly 2060 as explained in more detail below.

As best shown in Fig. 16A, the knife lockout assembly 2060 includes an over the top knife throw linkage 2061 having bifurcated flanges 2062a and 2062b at a distal end thereof and a catch 2064 at a proximal end thereof. Bifurcated flanges 2062a and 2062b are configured to operably engage respective upper flanges 2072a and 2072b of the trigger assembly 2070 about a pin 2065 such that proximal movement of the trigger tab 2071 about pivot 2077 forces the knife throw linkage 2061 distally to advance the knife 190 (not shown in Fig. 16A). Catch 2064 at the proximal end of the knife throw linkage 2061 is configured to operably interface with a flange 2084 of a knife lockout bar 2080 when the handle 2040 is not fully actuated as explained in more detail below.

More particularly, knife lockout bar 2080 includes flange 2084 at a proximal end thereof and a pivot arm 2081 that extends therefrom having pivot mount 2082 that interfaces with a pivot 2021 disposed within the forceps housing 2020. A distal end 2083 of the lockout bar 2080 is configured to operably couple to a proximal tab 2037 disposed at the proximal end of the drive assembly 2030.

When movable handle 2040 is disposed in an open or spaced position relative to fixed handle 2050, the trigger assembly 2070 (e.g., trigger tab 2071) is passively prevented from being actuated. In other words, the movable handle 2040 blocks or prevents the trigger tab 2071 from being actuated to advance the knife 190. In addition, when handle 2040 is disposed in a open configuration relative the fixed handle 2050, the drive assembly 2030 is biased in a proximal-most position which, in turn, biases the jaw members 110 and 120 in an open position as explained above. When the drive assembly 2030 is biased in a proximal-most position, the proximal tab 2037 is generally aligned along longitudinal axis A-A through the forceps 2000 and flange 2084 is positioned in operative, blocking engagement against catch 2064 to prevent advancement of the knife throw linkage 2061. As can be appreciated, this arrangement acts as a second safety lockout to prevent accidental advancement of the knife through tissue.

When handle 2040 is actuated and move proximally toward handle 2050, the passive lockout feature mentioned above no longer prevents actuation of the trigger assembly 2070, however, the knife lockout assembly 2060 remains in an engaged position to prevent advancement of the knife 190. When the handle 2040 is fully engaged and the drive assembly 2030 is fully actuated, the proximal tab 2037 of the drive assembly 2030 is cammed to engaged the distal end 2083 of the lockout bar 2080 which, in turn, pivots the proximal flange 2084 of the lockout bar 2080 out of engagement with the catch 2064 of the knife throw linkage 2061 (See Fig. 16B).

It is important to note that the proximal tab 2037 is only cammed when the movable handle 2040 and the drive assembly 2030 are fully actuated therefore the knife 190 can not be advance without the jaw members 110 and 120 being fully closed. As a result thereof, tissue disposed between the jaw members 110 and 120 cannot be severed unless the jaw members 110 and 120 are disposed in a fully closed position.

Upon release of the trigger tab 2071, a knife spring 2067 (See Figs. 15D or 16B) returns the knife throw linkage 2061 to a proximal-most position allowing re-engagement of the catch 2064 with the proximal flange 2084 of the lockout bar when the handle 2040 is re-opened. More particularly, proximal tab 2037 releases distal end 2083 that allows the proximal flange 2084 of the knife bar 2080 to re-engage the catch 2064. A spring (not shown) may be utilized to facilitate re-engagement of the proximal flange 2084 of the knife bar 2080 with catch 2064 or upon release of the movable handle 2040, the proximal tab 2037 may be configured to cam the proximal flange 2084 of the knife bar 2080 back into engagement with the catch 2064.

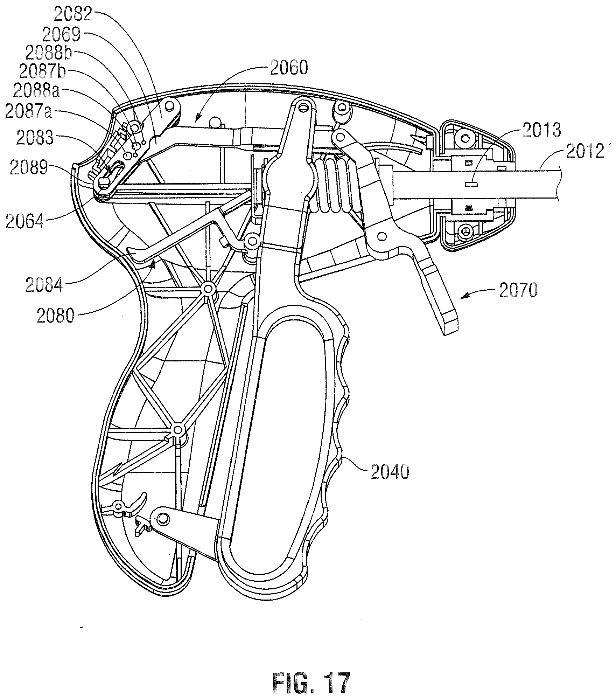

Fig. 17 shows an extension link 2082 that is configured to operably engage the knife lockout assembly 2060 and adjust the throw (e.g., distance of knife travel) of the knife 190 depending upon the jaw configurations and the length of the knife channel disposed therebetween. For example, the knife throw 2061 includes a proximal end 2069 that is dimensioned to engage a corresponding aperture 2087a or 2087b disposed in the extension link 2082. Depending upon the what aperture 2087a or 2087b the manufacturer engages during assembly will determine the ultimate throw distance of the knife 190. In one embodiment, the knife shaft 2012' and the extension link 2082 may include visual indicia or color codes 2088a and 2088b to facilitate assembly, e.g., the shaft 2012' may include a color code 2013 on a side thereof that corresponds to a color code 2088a on one of the apertures 2087a to reflect the required throw distance for the knife for a particular shaft 2012'. Different shafts would have different color codes to facilitate assembly. A proximal end 2089 of the extension link 2082 may include an elongated slot 2083 that receives the catch 2064 of the knife assembly 2060. Catch 2064 and the above-described knife bar 2080 operate in a similar manner as described above.

From the foregoing and with reference to the various figure drawings, those skilled in the art will appreciate that certain modifications can also be made to the present disclosure without departing from the scope of the same. For example, it may be preferable to add other features to the forceps, e.g., an modular articulating assembly to axially displace the end effector assembly relative to the elongated shaft.

It is also contemplated that the forceps (and/or the electrosurgical generator used in connection with the forceps) may include a sensor or feedback mechanism (not shown) that automatically selects the appropriate amount of electrosurgical energy to effectively seal the particularly-sized tissue grasped between the jaw members. The sensor or feedback mechanism may also measure the impedance across the tissue during sealing and provide an indicator (visual and/or audible) that an effective seal has been created between the jaw members.

As can be appreciated, locating the switch on the forceps has many advantages. For example, the switch reduces the amount of electrical cable in the operating room and eliminates the possibility of activating the wrong instrument during a surgical procedure due to "line-of-sight" activation. Moreover, it is also envisioned that the switch may be configured such that it is mechanically or electro-mechanically decommissioned during trigger activation to eliminate unintentionally activating the device during the cutting process. It is also envisioned that the switch may be disposed on another part of the forceps, e.g., the fixed handle, rotating assembly, housing, etc. In one embodiment, the switch (or another switch) may also be configured to control the knife assembly, e.g., the knife assembly may be coupled to the same or alternate electrosurgical energy source to facilitate cutting of the tissue.

It is also envisioned that the forceps may be equipped with an automatic, electro-mechanical release mechanism (not shown) that releases the tissue once an end seal is determined (i.e., end-tone signal from the generator). For example, an electromechanical interface may be configured to automatically release the t-shaped pin of the movable handle from catch basin of the fixed handle upon an end tone condition.

It is also contemplated that the forceps may be dimensioned to include a trigger assembly that operates in lieu of the switch assembly to activate the forceps to seal tissue while also advancing the knife to divide the tissue across the seal. For example, the trigger assembly could be configured to have two stages: a first or initial stroke stage that activates the generator to selectively seal tissue; and a second or subsequent stage that advances the knife through the tissue. Alternatively, another embodiment may include a trigger assembly that simultaneously activates the jaw members and to seal tissue and advances the knife through the tissue during activation. The trigger assembly may also be configured to move the knife assembly (or one or more of the components thereof) proximally to cut tissue disposed between the jaw members.

It is also contemplated that the rotating assembly may be equipped with one or more mechanical interfaces that are rotatable with or within the rotating assembly and that are configured to produce tactile and/or audible feedback to the user during rotation. The tactile and/or audible feedback (i.e., a "click") may be configured to correspond to a particular degree of rotation of the end effector assembly about the axis A-A. It is also contemplated that one or more types of visual indicia may also be employed with the rotating assembly to correspond to the amount or degree of rotation of the end effector assembly and may be designed correspond to or relate to the audible and/or tactile feedback depending upon a particular purpose.

It is also envisioned that the forceps may be configure to include a visual indicator (which cooperates with the "end tone" indicator on the generator) to provide visual confirmation of a successful seal (e.g., a green LED indicator). The visual indicator (not shown) may be employed on or in connection with the end effector assembly or shaft which is in line-of-site of the surgeon during use. The visual indicator may also be designed to warn the user of a mis-seal condition or a re-grasp condition (e.g., a red LED indicator). Alternatively, the visual indicator may also be configured to provide progressive feedback of the formation of the seal during the sealing process. For example, a series of LEDs may be employed on the end effector assembly (or shaft) that progressively illuminate through the sealing process to provide visual feedback to the user regarding the status of the seal.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto.

## Claims

1. A modular shaft assembly for use with a variety of different endoscopic forceps (10) each having a housing (20) including a handle assembly (30) and at least one moveable handle (40), the modular shaft assembly comprising:
a shaft (12) having proximal and distal ends; and
an end effector assembly (100) including a pair of jaw members (110; 120) attached to the distal end of the shaft and a universal drive assembly (130) attached at the proximal end of the shaft, wherein:
said universal drive assembly is operably engageable with the handle assemblies of the variety of different endoscopic forceps such that actuation of the at least one movable handle of any of the variety of different forceps causes the universal drive assembly to actuate the jaw members of the end effector assembly to move between an open position wherein the jaw members are disposed in spaced relation relative to one another to a closed position for grasping tissue therebetween, said modular shaft assembly being **characterized in that** the shaft includes a plurality of mechanical interfaces that are configured to mate with a corresponding common plurality of mechanical interfaces disposed within the variety of endoscopic forceps;
the plurality of mechanical interfaces that are configured to mate with a corresponding common plurality of mechanical interfaces disposed within the variety of endoscopic forceps are disposed on an outer periphery of the shaft;
the shaft includes a bushing (1150) disposed on the shaft that mates within a corresponding mechanical interface disposed within a distal end the housing;
the shaft includes a rotating assembly (1080) affixed atop the shaft for rotating the shaft relative to the handle assembly with which it is engaged;
the shaft includes a knife rod (193) disposed in said shaft (12) and a knife actuator (1075) that slideably mounts atop a midway portion (1014) of the shaft in between the rotating assembly and the bushing; and
an elongated slot (1015) is defined within the midway portion of the shaft and cooperates with the knife actuator such that said knife actuator (1075) is adapted to advance and retract the knife rod.

2. A modular shaft assembly according to claim 1 wherein the distal end of the shaft includes a universal coupling for selectively engaging a corresponding coupling on a variety of end effector assemblies having a variety of different jaw member configurations.

3. A modular shaft assembly according to claim 1 or 2, wherein the bushing includes a series of mechanical rings (1151; 1152) that cooperatively engage a series of corresponding groves (1221; 1223) provided in the housing for mounting and securing the shaft within the forceps housing.

4. A modular shaft assembly according to any preceding claim, wherein the knife actuator includes a series of apertures (1076a; 1076b) disposed therealong for engagement with a pin (1262) of a knife stroke link (1260) facilitating the adjustment of a knife stroke length, the knife stoke link operatively engaging the knife actuator and a trigger assembly (70) of the housing for actuating the knife actuator.

## Patentansprüche

1. Modulare Schaftanordnung zur Verwendung mit einer Vielzahl von verschiedenen endoskopischen Zangen (10), die jeweils ein Gehäuse (20) haben, das eine Handgriffbaugruppe (30) und mindestens einen beweglichen Handgriff (40) aufweist, wobei die modulare Schaftanordnung umfasst:
einen Schaft (12) mit proximalen und distalen Enden; und
eine Endeffektoranordnung (100), die ein Paar von Klemmbackenelementen (110; 120), die an dem distalen Ende des Schafts befestigt sind, und eine universelle Antriebsanordnung (130) aufweist, die an dem proximalen Ende des Schafts befestigt ist, wobei:
die universelle Antriebsanordnung mit den Handgriffbaugruppen von der Vielzahl von verschiedenen endoskopischen Zangen betriebsfähig in Eingriff bringbar ist, so dass eine Betätigung von dem mindestens einen beweglichen Handgriff von einer von der Vielzahl von verschiedenen Zangen bewirkt, dass die universelle Antriebsanordnung die Klemmbackenelemente der Endeffektoranordnung betätigt, um sich von einer offenen Position, bei der die Klemmbackenelemente in beabstandeter Beziehung relativ zueinander angeordnet sind, in eine geschlossene Position zum Ergreifen von Gewebe dazwischen zu bewegen, wobei die modulare Schaftanordnung **dadurch gekennzeichnet ist, dass**
der Schaft eine Vielzahl von mechanischen Schnittstellen aufweist, die konfiguriert sind, um sich mit einer korrespondierenden gemeinsamen Vielzahl von mechanischen Schnittstellen, die in der Vielzahl von endoskopischen Zangen angeordnet sind, zu fügen;
die Vielzahl von mechanischen Schnittstellen, die konfiguriert sind, um sich mit einer korrespondierenden gemeinsamen Vielzahl von mechanischen Schnittstellen, die in der Vielzahl von endoskopischen Zangen angeordnet sind, zu fügen, an einem Außenumfang des Schafts angeordnet sind;
der Schaft eine an dem Schaft angeordnete Hülse (1150) aufweist, die sich in eine korrespondierende mechanische Schnittstelle, die in einem distalen Ende des Gehäuses angeordnet ist, fügt;
der Schaft eine Drehanordnung (1080) aufweist, die oben auf dem Schaft befestigt ist, um den Schaft relativ zu der Handgriffbaugruppe, mit der er in Eingriff steht, zu drehen;
der Schaft eine Messerstange (193), die in dem Schaft (12) angeordnet ist, und einen Messerbetätiger (1075) aufweist, der oben auf einem Mittelabschnitt (1014) des Schafts zwischen der Drehanordnung und der Hülse befestigt ist; und
ein langgestreckter Schlitz (1015) in dem Mittelabschnitt des Schafts definiert ist und mit dem Messeraktuator zusammenwirkt, so dass der Messeraktuator (1075) angepasst ist, um die Messerstange vorzuschieben und zurückzuziehen.

2. Modulare Schaftanordnung nach Anspruch 1, wobei das distale Ende des Schafts eine universelle Kopplung für einen wahlweise Eingriff mit einer korrespondierenden Kopplung an einer Vielzahl von Endeffektoranordnungen mit einer Vielzahl von verschiedenen Klemmbackenelementkonfigurationen aufweist.

3. Modulare Schaftanordnung nach Anspruch 1 oder 2, wobei die Hülse eine Reihe von mechanischen Ringen (1151; 1152) aufweist, die mit einer Reihe von korrespondierenden Rillen (1221; 1223) zusammenwirkend in Eingriff stehen, die in dem Gehäuse zum Befestigen und Sichern des Schaftes in dem Zangengehäuse bereitgestellt sind.

4. Modulare Schaftanordnung nach einem vorstehenden Anspruch, wobei der Messeraktuator eine Reihe von Öffnungen (1076a; 1076b) aufweist, die dort entlang für einen Eingriff mit einem Zapfen (1262) einer Messerhubverbindung (1260), die die Einstellung einer Messerhublänge ermöglicht, angeordnet sind, wobei die Messerhubverbindung mit dem Messeraktuator und einer Auslöseanordnung (70) des Gehäuses betriebsfähig in Eingriff steht, um den Messeraktuator zu betätigen.

## Revendications

1. Ensemble d'arbre modulaire pour utilisation avec une variété de différents forceps endoscopiques (10) ayant chacun un boîtier (20) comprenant un ensemble de poignée (30) et au moins une poignée mobile (40), l'ensemble d'arbre modulaire comprenant :
un arbre (12) ayant des extrémités proximale et distale ; et
un ensemble d'effecteur terminal (100) comprenant une paire d'éléments de mors (110; 120) fixée à l'extrémité distale de l'arbre et un ensemble d'entraînement universel (130) fixé à l'extrémité proximale de l'arbre, dans lequel :
ledit ensemble d'entraînement universel peut être engagé en service avec les ensembles de poignée de la variété de forceps endoscopiques différents de sorte que l'actionnement de la au moins une poignée mobile de l'un quelconque de la variété de différents forceps amène l'ensemble d'entraînement universel à actionner les éléments de mors de l'ensemble d'effecteur terminal pour se déplacer entre une position ouverte, dans laquelle les éléments de mors sont disposés en relation espacée mutuelle et une position fermée pour saisir un tissu disposé entre eux, ledit ensemble d'arbre modulaire étant **caractérisé en ce que** :
l'arbre comprend une pluralité d'interfaces mécaniques qui sont configurées pour s'accoupler à une pluralité commune correspondante d'interfaces mécaniques disposées dans la variété de forceps endoscopiques ;
la pluralité d'interfaces mécaniques qui sont configurées pour s'accoupler à une pluralité commune correspondante d'interfaces mécaniques disposées dans la variété de forceps endoscopiques est disposée sur une périphérie externe de l'arbre ;
l'arbre comprend une douille (1150) disposée sur l'arbre et qui s'accouple dans une interface mécanique correspondante disposée dans une extrémité distale du boîtier ;
l'arbre comprend un ensemble rotatif (1080) fixé au sommet de l'arbre pour faire tourner l'arbre par rapport à l'ensemble de poignée avec lequel il est engagé ;
l'arbre comprend une tige à couteau (193) disposée dans ledit arbre (12) et un actionneur de couteau (1075) qui est monté à coulissement au sommet d'une partie centrale (1014) de l'arbre entre l'ensemble rotatif et la douille ; et
une fente allongé (1015) est définie dans la partie centrale de l'arbre et coopère avec l'actionneur de couteau de sorte que ledit actionneur de couteau (1075) soit à même de faire avancer et de rétracter la tige à couteau.

2. Ensemble d'arbre modulaire selon la revendication 1, dans lequel l'extrémité distale de l'arbre comprend un couplage universel pour s'engager sélectivement sur un couplage correspondant d'une variété d'ensembles d'effecteurs terminaux ayant une variété de différentes configurations d'éléments de mors.

3. Ensemble d'arbre modulaire selon la revendication 1 ou 2, dans lequel la douille comprend une série de bagues mécaniques (1151; 1152) qui s'engagent par coopération sur une série de rainures correspondantes (1221 ; 1223) ménagées dans le boîtier pour monter et fixer l'arbre dans le boîtier du forceps.

4. Ensemble d'arbre modulaire selon l'une quelconque des revendications précédentes, dans lequel l'actionneur de couteau comprend une série d'ouvertures (1076a ; 1076b) disposées le long de celui-ci pour s'engager sur une broche (1262) d'une liaison de course de couteau (1260) facilitant l'ajustement d'une longueur de course de couteau, la liaison de course de couteau s'engageant en service sur l'actionneur de couteau et un ensemble de gâchette (70) du boîtier pour actionner l'actionneur du couteau.
